(19) **Europäisches Patentamt**

**European Patent Office**

**Office européen des brevets**

(11) **EP 0 632 730 B1**

(12) **EUROPEAN PATENT SPECIFICATION**

(45) Date of publication and mention
of the grant of the patent:
**11.10.2000 Bulletin 2000/41**

(21) Application number: **93909157.5**

(22) Date of filing: **24.03.1993**

(51) Int. Cl.[7]: **A61L 9/01**, C02F 3/04,
B01D 53/00

(86) International application number:
**PCT/US93/02733**

(87) International publication number:
**WO 93/18800 (30.09.1993 Gazette 1993/24)**

(54) **IMMOBILIZED FILM- BIOREACTOR**

IMMOBILISIERTER FILM-BIOREAKTOR

BIOREACTEUR A FILMS IMMOBILISES

(84) Designated Contracting States:
**AT BE CH DE DK ES FR GB GR IE IT LI LU MC NL
PT SE**

(30) Priority: **25.03.1992 US 857406**

(43) Date of publication of application:
**11.01.1995 Bulletin 1995/02**

(73) Proprietors:
• **BISHOP, Dolloff F.**
**Cincinnatti, OH 45244 (US)**
• **GOVIND, Rakesh**
**Cincinnati, OH 45221 (US)**

(72) Inventors:
• **BISHOP, Dolloff F.**
**Cincinnatti, OH 45244 (US)**

• **GOVIND, Rakesh**
**Cincinnati, OH 45221 (US)**

(74) Representative:
**Harding, Charles Thomas et al
D. Young & Co.
21 New Fetter Lane
London EC4A 1DA (GB)**

(56) References cited:
| | |
|---|---|
| **EP-A- 0 224 889** | **EP-A- 0 300 101** |
| **EP-A- 0 470 468** | **DE-A- 2 643 211** |
| **DE-A- 3 423 285** | **DE-A- 3 607 864** |
| **DE-A- 4 017 384** | **US-A- 2 188 162** |
| **US-A- 2 200 580** | **US-A- 4 179 374** |
| **US-A- 4 242 448** | **US-A- 4 544 381** |
| **US-A- 4 680 111** | **US-A- 4 681 851** |
| **US-A- 4 762 612** | **US-A- 4 806 148** |
| **US-A- 5 037 688** | **US-A- 5 085 766** |

## Description

### Field of the Invention:

This invention involves biodegradation and/or biotransformation of volatile contaminants in the gas phase under controllable aerobic and/or anaerobic conditions. The invention has broad application for removing volatile, harmful contaminants in the gas phase.

### Background of the Invention:

Recently there has been increased interest in the removal of organic and inorganic contaminants present in low concentrations from gas streams. Conventional technologies that have been used industrially for organics include (1) adsorption onto porous substances such as activated carbon; (2) absorption into liquid medium followed by stripping of the liquid; (3) combustion or incineration; and (4) pervaporation using selective membranes to selectively remove the harmful volatiles. Lime treatment is used in scrubbers to remove sulfur oxides from stack gases. Chemical addition and reaction is based on control of nitrogen oxides. These technologies present several problems, including disposal of generated wastes (for example, calcium sulfate, unused lime and spent activated carbon), cost of equipment, materials and energy required for operation.

While biological treatment of aqueous waste streams has been an established practice, the use of biofilters for treatment of air or gaseous streams has not been extensively investigated in the United States. Pomeroy and Carlson have reported an approach for removal of sewage-related odors (Pomeroy, R.D., "Controlling Sewage Plant Odors". Consulting Engineers Vol 20: (1963) p. 101; Carlson, et al, "Soil Beds for the Control of Sewage Odors", J. Water Pollution Control Federation, Vol. 38: No. 5 (1966) 829-840. The primary mechanism for removal of the odorous compounds such as hydrogen sulfide, mercaptans, terpenes, amines, etc, may have been adsorbed on support material. There was no disclosure of biodegradation. It is, furthermore, known that soil beds are effective in removing sulfur-containing gasses and can serve as a sink for hydrocarbons.

In more recent years, methods for use of biofilters have been disclosed in Europe. It has been found that an important operating parameter is the moisture content of the filter bed, which plays an important part in determining removal efficiency. It has also been determined that the most active microbes in a biofilter are the heterotrophic and chemoorganotrophic groups. Actinomycete spp. are particularly useful since they can act on a wide variety of organic compounds and are capable of killing pathogens under some conditions.

Conventional biofilter technology using soils and peat or compost have been widely used in Europe with more than 500 field applications for control of volatile organic compounds (VOCs) and odors. These applications usually involve low VOC concentrations (<300 ppm). In this technology, the void space in the filter media controls the air flow rates through the biofilter. Soil biofilters with low void space have low air flows and generally use up to 15 minutes of air retention time. Peat or compost biofilters, called biotower, with higher bed void space usually require less than two minutes of air retention time. Typical pressure losses across the filters range from less than 498 to 2988 N/m$^2$ (2 to 12 inches of water). Solid nutrients and buffers such as limestone are incorporated in the media material The contaminated air is prehumnidified to supply needed moisture. Additional water maybe sprayed intermittently on the top of the bed. For VOC control, the start-up acclimation typically requires about one month to reach full removal of VOCs. Filters operating at low VOC concentrations are reported to remove VOC's for several (1 to 5) years before the filter media is replaced and the filter operation reacclimated.

It is known that the distribution of the microbes in a biofilter may vary at different parts of the filter. Population density is usually highest at the gas entrance of the biofilter, where the microbes preferentially metabolize the more readily degradable influent compounds. In prior art systems, the slowly degradable compounds biodegraded in all portions of the biofilter. However, the upper portion of the biofilter became under-loaded as to amount of biomass since the microbes at the lower levels consume more of the biodegradable compounds. In the conventional fine particle filter this situation of uneven distribution of biomass can gave rise to channelling with inefficiency and possible break-through of the less degradable compounds. In addition, it is not possible to remove the gradually increasing biomass and replenish the decreasing nutrients. Eventually the media of these biofilters must be replaced.

A biofilter system, termed the BIKOVENT system, consisting of prefabricated concrete parts which form an aeration plate to give uniform air distribution combined with drainage ducts was developed by Drs. Hans Gethke and Detlef Eitner of Aachen, West Germany. The BIKOVENT system has been used extensively in West Germany and Austria for odor control and for controlling volatile organic compounds (VOCs) in waste air streams. The technology has recently been introduced into the United States by Biofiltration, Inc., Gainsville, Florida.

A second conventional biofilter, the BIOTON filter using peat or compost support in a biotower configuration has also been developed in Europe. The BIOTON technology offered by Clair Tech, b.v. of The Netherlands has also been introduced into the United States by Ambient Engineering, Inc., Matawan, New Jersey. These conventional biofilters present a major disadvantage in that biomass build-up occurring in the biofilter with time cannot be removed. Such buildup leads to filter plugging and ultimately requires replacement of the media, forcing process shutdown.

U.S. patent 2,200,580 discloses use of a method of treating fluid to control odors. No method of biotransformation or biodegradation of gases is taught therein. Schulhoff (U.S. Patent 2,188,162) teaches an aerobic biofilter having tubes for the treatment of liquids. The instant invention requires higher pH both for degradation and biotransformation.

## Summary of the Invention:

The biofilters of the invention consist of three basic components: (1) a solid media incorporated in a bed supports microorganisms capable of degrading organics and biotransforming inorganics; (2) a liquid trickling film which keeps the organisms moist while supplying them with nutrients and buffers; and (3) a gaseous stream containing volatile organic or inorganic contaminants which is contacted with the support material. A variety of material can be used as the support material. The support material may take a variety of forms, such as hollow tubes or fibers. All supports are made of wettable material and surfaces of preferred supports are porous. The supports must be such that microorganisms can adhere thereto. The supports may be made of hydrophilic plastics. The principle advantage of this invention is that it allows removal of excess biomass build-up and excessive pressure drop in the filter. Thus the media does not need to be replaced and uninterrupted operation of the filter is permitted. Biofilters of the invention also may use a combination of anaerobic and aerobic organisms by creating differing environments (nitches) in appropriate sequences within the biofilter.

## Brief Description of the Figures:

Fig. 1 is a drawing of the biofilter with heat jacket.
Fig. 2(a) is a drawing of a cross section of a straight passage support media.
Fig. 2(b) is a drawing of a cross section of a filter of extruded ceramics
Fig. 3 depicts a biofilm reactor.
Fig. 3(a) depicts biofilm with entering gas flow on the side of the biomass.
Fig. 3(b) depicts biofilm with entering gas flow on the side of the film opposite the biomass.
Fig. 4 shows a biofilter support in a corrugated arrangement.

## Detailed Description of the Invention:

Biofilters for treating contaminated air use immobilized biofilms on filter support media to biodegrade sorbed VOCs and biotransform inorganic oxides to sulfur ($SO_x$) and nitrogen ($NO_x$) to residuals such as carbon dioxide, water and various acids ($HCl$, $H_2SO_4$, $HNO_3$). The buffers in the trickling liquid neutralize the acid. Some preferred support materials include straight passage media such as extruded ceramics and extruded carbon. Straight passage supports permit removal of excess biomass by hydraulic or chemical cleaning and minimize pressure loss. The biofilters are characterized by low pressure drop across the bed, good contact of influent gas with the biomass, and satisfactory biomass adherence to the support. The biofilters are easily cleaned of excess biomass or are self-cleaning.

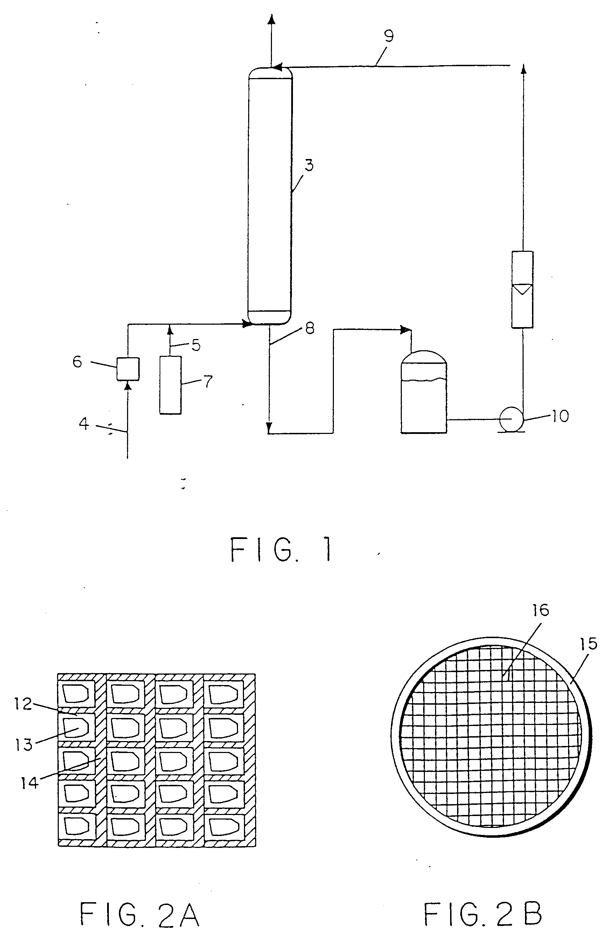

A particularly useful material for use in accord with the teachings of the invention are thin-walled extruded ceramic materials such as those used in catalytic converters in cars. These extruded ceramic provides high void fractions with thin walls which provide very large surface area to hold large amounts of biomass per unit volume of reactor. Such materials minimize the size of the biofilter. Such a filter is seen at **Fig. 2(b)** wherein 15 is the side of the column and 16 indicates a wall of the extruded ceramic.

The biofilters of the invention are adaptable for use in controlling sulfur and nitrogen oxides in contaminated gases. The packed filters of the invention can effectively be used to reduce acid rain. The invention, when adapted to $SO_x$ and $NO_x$ control, also provides for biological transformation of sulfur and nitrogen oxides ($SO_x$,$NO_x$) to sulfur and nitrogen gas with recovery of the sulfur and release of the nitrogen gas into the atmosphere as described subsequently.

A particularly important aspect of the invention is the preparation of the filter with organisms particularly effective for the degradation of the target contaminant. For example, a specifically adapted biomass may be prepared by bubbling stack gasses containing sulfur and nitrogen oxides through a liquid phase reactor to provide organisms particularly adapted to oxidize sulfur and nitrogen oxides in the stack gas.

The bioprocess, using microbial degradation, requires nutrients, water, sufficient buffer for pH control, and, for a few VOCs, selected co-metabolites to destroy VOCs. It is essential that the surfaces supporting the biomass be wettable. The support material should be porous so that the liquid nutrients can be evenly distributed by capillary action and the pores provide areas protected from hydrodynamic shear forces so that the microorganisms can attach initially.

For optimal attachment the following characteristics are preferred:

(1) Size of straight passages can vary from 25 straight passages per square inch (superficial surface area per unit volume = 20 inch$^2$/1 inch$^3$) to 400 straight passages per inch. (80 inches$^2$/1 inch$^3$); (1 inch = 2.54 cm)
(2) bed void fraction >0.3 but typically >0.6; (3) open pores to allow free distribution of water in support material; (4) electrically neutral surface; (5) Hydrophilic surface; (6) shear stress due to liquid flow less than 5.0 N/m$^2$; (7) high porosity of support media; and (8) media surface roughness with peaks/valleys exceeding 0.05 micrometres.

Gas entering the biofilter may be humidified or water may be sprayed intermittently in the biofilter in order to prevent drying of the column. Variations in the contaminant concentrations are accommodated by control of contaminant transport from the air to the biofilm, sorptive capacity of the biofilm, and adsorption of filter media. In most instances, the contaminants are transported as gases under low pressure to the biofilter which is kept constantly moistened with a liquid film which keeps the microorganisms moist and provides necessary nutrients and buffers to the system. In practice of the invention, buffer and nutrients may also be incorporated as solids in the filter media or added to the filters in liquid streams entering at appropriate levels of the filter. Moisture may also be added by prehumidification of the contaminated air or alternatively as aqueous spray into the biofilter. Needed co-metabolites may be added to the recirculating liquid stream. The process of the invention requires modest amounts of energy to move air though the biofilter.

The transport of the organics to the reactive sites may occur in several different ways. Transport of the organic from the gas phase to the solid support and subsequent adsorption/absorption of the organic material to the support with subsequent exposure to the biofilm is frequent when porous materials are used. Such porous support materials as activated charcoal may be particularly useful when at least one VOC is readily adsorbed. The organic may be held on and in the support. Organisms of the biofilm may then act on the organic compound which has been removed from the air stream as the organic compound becomes available on passage from the support material. In some instances, the organics may be selectively absorbed from the air flow into the support to a biofilm on the opposite side of the support. This may occur, for example, when the support is a straight path porous tube with biofilm on one side of the support wherein moisture flows down the biofilm but does not contact the incoming air stream containing the contaminant. The biodegradation in the biofilm on the Opposite side of the support may be anoxic or anaerobic.

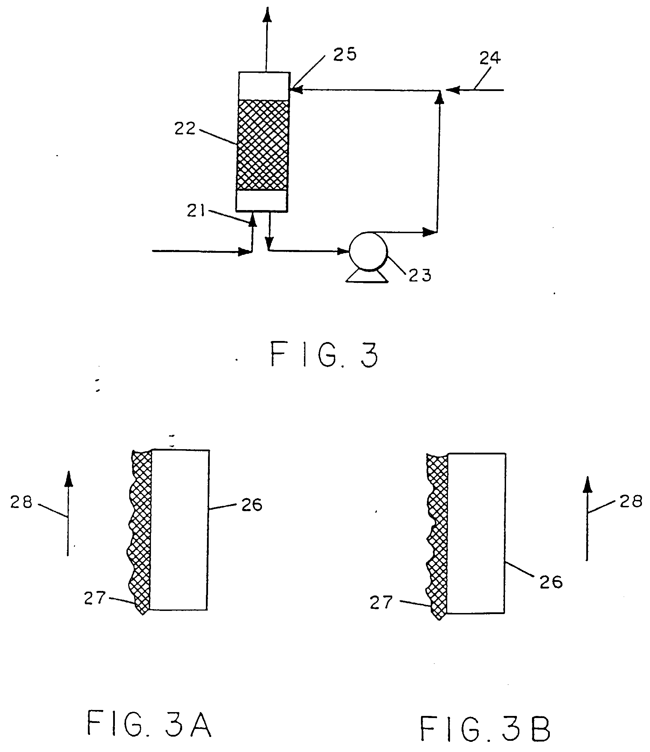

**Fig. 3** depicts a biofilm reactor wherein the gas enters the chamber with the immobilized film bed (22) at the gas inlet (21). The media with nutrients enters the filter from above (25) and passes through the filter to the a pump (23) which pumps the nutrient-containing fluid for recycling. Fresh nutrients may be added in the line (24) to replenish the supply. **Fig. 3(a)** shows the support media (26) with biomass (27) wherein the gas flow (28) flows on the side of the biomass. **Fig. 3(b)** shows the same filter with the gas flow (28) on the side of the porous media support and on the side opposite the biofilm.

In other instances, the biofilter may be arranged so that the organic or inorganic contaminant in the gas phase is transported to the liquid film with subsequent dissolution of the contaminant into the aqueous phase. The contaminants may then be acted on directly by the biofilm or may be transported to a solid support with subsequent adsorption/absorption, then back diffused from the support material onto the biofilm with subsequent degradation. In most instances, the organics or inorganics diffuse through the biofilm with concurrent oxidative degradation or biotransformation. The liquid stream runs countercurrent to the gaseous stream. Countercurrent flow provides the most efficient use of the biofilter.

Organic and inorganic chemicals contaminating air that may be profitably exposed to the process include petroleum chemicals, solvents (chlorinated and un chlorinated aliphatic and aromatic compounds), industrial chemicals (alcohols, ketones, etc.), organo-sulfur compounds, $SO_x$ and $NO_x$. The process of the invention can be used when concentrations of the VOCs or organic chemical in air or gas phase range from a trace to thousands of ppm. The use of the biofilter to remove sulfur oxides ($SO_x$) and/or nitrogen oxides ($NO_x$) from stack gases has never been taught.

Biofilters of the instant invention differ from conventional technologies in several ways. The biofilters in accord with this disclosure provide wide flexibility to maximize efficacy and to lower cost of operation in removal of volatile organic compounds (VOCs). In addition, these biofilters can be adapted to remove sulfur and nitrogen oxides ($SO_x$ and $NO_x$) from stack gases. In accord with the teachings disclosed herein, various organisms, including both aerobic and anaerobic organisms, can be used. Mixed cultures that have been acclimated to specific organics can be used in accord with the teachings herein. Pure cultures which are particularly useful for degrading specific organics may also be used. By the methods taught herein aerobic and anaerobic organisms may be used sequentially to degrade a mixture of organics containing components that are recalcitrant under some conditions. In some instances, the vertical stratification of the microorganisms with different predominant organisms existing at various levels of the biofilter may occur through the process of natural selection. In other instances, microorganisms of a certain type may flourish at a specific locational niches which maximizes their growth due to the existence of controlled optimum conditions, such as type or concentration of co-metabolites and nutrients, pH, temperature, and oxidation/reduction potential.

The continuous, controlled degradation which can be attained in accord with the teachings of this disclosure prevent break-through of the contaminants. While initial concentration of the contaminants in the support material and biofilm will increase initially, when a steady-state is established the rate of transport of the contaminants from the gas phase to the support material becomes balanced by the rate of biodegradation or biotransformation of the contaminants and by cell production. A high rate of bioactivity is possible because of the existence of the immobilized biofilm, which can contain a significantly higher concentration of the microorganisms than that found in liquid phase conventional activated sludge. Since the rate of biodegradation is dependant on the concentration of the microorganisms, a significantly high concentration in the biofilm will result in an increased rate of biodegradation and biotransformation.

By the methods of the invention, unlike in aqueous waste biotreatment, it is possible to avoid contamination of the support material by nonbiodegradable organics or high molecular weight contaminants which cause problems in completely mixed continuous systems, such as in activated sludge plants or fluidized bed reactors which handle aqueous waste streams. Since the organic contaminants are introduced into the biofilter through the gas phase, high molecular weight materials or entrained particles can be removed by pretreatment such as water spraying, and are not passed into the biofilter.

The support material is in a straight passage arrangement in the form of a hollow tube or a fiber. When the support material is in a straight passage arrangement allowable parameters for the liquid and gas flows are flexible, since there is less likelihood of flooding.

The shape chosen for any biofilter will depend on the space available and nature of the gas stream. For example, filters having square, rectangular, triangular or circular cross-sections may be used. The functional differences will primarily exist between those that have a cross-sectional area that remains constant throughout the height of the column and those wherein the cross-sectional area increases with height. For example, the biofilter bed may be cylindrical or conical, while a bed having a triangular cross-section may have the same cross-section dimensions throughout its length or may be shaped like an inverted pyramid. Designs such as conical or inverse pyramidal design will result in increased residence time of the gas stream in the upper portion of the bed due to lower superficial gas velocity, thereby allowing the slower degrading organics to be removed from the gas stream.

Supports in straight passages may have segments that act as niches ("shelters") for microorganisms. The organisms growing on the support material can be varied from location to location. When the organisms are less robust, some of the organisms may be more able to survive and effect transformation of contaminants when they are sheltered in the niches

The microorganisms may be immobilized in polymeric matrices that allow diffusion of the target organic contaminant, but effectively block other organics present in the gas stream from inhibiting the biodegradation process and/or from poisoning the reaction. In some instances, the support materials may be gels that immobilize microorganisms. (See Example 4.)

The design of the biofilter is based on two important considerations: (1) pressure drop in the biofilter for the gaseous stream and (2) percentage removal of the contaminant due to biodegradation or biotransformation. The pressure drop will increase as the air velocity is increased in a bed of a given void fraction.

For removal of volatile organics tested in the examples, void fraction should be $\geq .1$ where void fraction is:

$$\frac{\text{total volume of void spaces}}{\text{total volume of the bed}}$$

Increased air velocity requires increased volume of the bed to maintain a fixed residence time and achieve a desired percentage of removal of the specific organics. The air velocity is also increased to respond to increased pressure drop. Since the primary energy cost in the operating system is related to cost of pumping the gas with the pollutant through the biofilter, increasing energy use in the filter results in increased operation cost. Therefore, to maximize total cost effectiveness the operating cost incurred and the depreciation cost of equipment must be taken into consideration in the design of a particular system.

The biofilter may be used in conjunction with a stripper to transfer the organics from the liquid waste stream to a gas stream. In that case, the stream used to strip the organics may be the air flow or may be a treated gas stream recycled from the biofilter. Nitrogen may be used as stripper and/or carrier when the system contains anaerobic bacteria.

In a biofilter, several different environments to support specific types of microbials at different locations in the filter may be created. As indicated, support structure and delivery of nutrients can be adapted so that different niches provide environments suitable for organisms that require special environments. Furthermore, species desired at a particular location can supported by injecting nutrients particularly needed by the target species. The addition of reagents into the environment in order to introduce specific levels of oxygenation of the environment at a given level makes it possible to have columns that have anaerobic growing conditions that induce anaerobic microbial growth in one location and aerobic growing conditions inducing growth of aerobic organisms at another location.

Biofilters of the invention may combine anaerobic and aerobic organisms by creating differing environment nitches in appropriate sequences within the biofilter. For example, when nitrogen is used for stripping and/or propelling the VOCs, the initial flow of air into the filter will support anaerobic organisms. Aerobic conditions may be created selectively at any level in the filter. One method involves injecting through the side of the biofilter a very dilute solution of hydrogen peroxide (3% w/v). Catalase, a terminal respiratory enzyme present in most aerobic living cells , breaks the hydrogen peroxide down into water and oxygen to create aerobic conditions at the site of the injection of the peroxide solution.

High concentration of VOCs (>300 ppm) in air produce substantial amounts of microbial growth in the biofilter. Technology of the invention using pelletized or straight passage supports permit removal of excess solids. Increasing biogrowth gradually causes filter plugging accompanied by excessive pressure losses. The pelletized media can be cleaned periodically. The cleaning may be achieved hydraulically or by use of chemical biocides followed by hydraulic washing. The straight passage configurations continuously release solids in the liquid stream leaving the biofilter. Hence, the straight passage filters may be self-cleaning.

A schematic of the biofilter system as shown in **Fig. 1**. It consists of a column **3** wherein carrier line **4** and substrate line containing the contaminant **5** feeds into the biofilter through gas inlet **8**. A flow meter on the carrier line **6** and a flow meter on the substrate line **7** allows the operator to control the amount and rate of VOC delivery to the bottom of the biofilter. Liquid containing nutrients flows down onto the biofilter from line **9** under the control of a pump **10**.

**Example 1: Straight Passage Celite Biofilter**

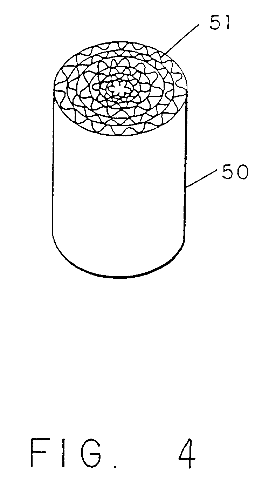

A straight passage celite biofilter was constructed using corrugated celite plates. A schematic of the biofilter is shown in **Fig.4**. The celite plates were arranged to provide vertical channels through which flow of air and liquid took place. The design and operating conditions for the biofilter are as follows:

| | |
|---|---|
| square column | 10 X 10 cm (4 X 4 inches) |
| column height | 90 cm |
| straight passage size | 6 mm X 6 mm |
| number of passages | 99 |
| gas flow rate | 600-1600 mL/min. |
| volume of biofilter | 8.77 liters |
| liq. nutrient flow rate | 1.5 liters/day |
| influent gas composition | |
| toluene | 1.774-4.731 mg/day |
| methylene chloride | 0.557-1.485 mg/day |
| TCE | 0.101-0.269 mg/day |
| chlorobenzene | 0.174-0.464 mg/day |
| ethylbenzene | 0.082-0.218 mg/day |
| total influent COD load | 0.768-2.048 gm/liter.day |
| ammonia loading | 75-150 mg/day |

The air flow rate was controlled by a thermal mass flow controller. The desired composition of the contaminants in air was obtained by injecting the liquid mixture of the compounds into the air stream using a syringe pump. The contaminated air stream flowed countercurrently to the trickling nutrient solution which was pumped to the top of the biofilter by a centrifugal pump. The composition of the nutrient solution is shown below:

| Salt | Concentration (mg/L) |
|---|---|
| $KH_2PO_4$ | 85.0 |
| $K_2HPO_4$ | 217.5 |
| $Na_2HPO_4$ | 266.4 |
| $NH_4Cl$ | 75.0 |
| $MgSO_4.7H_2O$ | 22.5 |
| $CaCl_2$ | 17.5 |
| $FeCl_2.5H_2O$ | .25 |
| Trace Elements: | |
| $MnSO_4.4H_2O$ | .0399 |
| $H_3BO_3$ | .0562 |
| $ZnSO_4.7.H_2O$ | .0428 |
| $(NH_4)_6Mo_7O_{24}$ | .0347 |
| FeCl.EDTA | .10 |
| Yeast Extract | .15 |

The nutrient solution exiting the bottom of the biofilter was drained.

As the startup procedure for the biofilter, biomass from a pilot scale activated sludge plant treating hazardous waste was suspended in an aerated aqueous phase bioreactor (column 100 mm diameter, 700 mm height). The bioreactor was fed daily with the VOC substrates. Nutrients consisting of a mixture of mineral salts, yeast extract and trace elements as indicated above were also added periodically. The biomass from the bioreactor was transferred to the biofilters by circulating the bioreactor suspension through the biofilter. The suspension was kept in the biofilter successfully attached to the support medium. The transfer of biomass from bioreactor suspension to the biofilter was confirmed by the fact that the bioreactor suspension drained from the biofilter at the end had lost its turbidity.

All of the effluent compounds except for TCE were completely mineralized.

Referring to Fig. 2, 12 refers to the biofilm, 13 refers to the flow channel through which the gas and fluid flow, and 14 refers to the support plate.

Air may be used as the source of additional oxygen to insure full biooxidation in the bioreactor. The soluble $SO_{4=}$ and $NO_{3-}$ ions in the aqueous stream exiting the oxidation reactor are bioreduced in an anaerobic/anoxic bioreactor reactor using a waste organic source such as sewage sludge to elemental sulfur, $H_2S$, and $N_2$. The alkalinity produced in the bioreduction is used to neutralize the acid produced in the biooxidation process by recycling the aqueous phase from the anaerobic/anoxic bioreactor to the first bioreactor. Any $H_2S$ produced in the bioreduction process is reduced to elemental sulfur. Carbon dioxide, acting as a co-substrate, is transformed in the bioreactor to biomass.

## Claims

1. A biofilter comprising:

(a) a bed having wettable straight passage supports with biomass adherent thereto, said passage supports being chosen from a plurality of hollow tubes, fibres, extruded material with multiple straight channel passages running vertically through said extruded material or corrugated plates arranged to provide vertical straight passages;
(b) an inlet for admission of a gaseous stream into the biofilter; and
(c) an inlet for a liquid stream positioned in such a manner that the liquid stream runs countercurrent to the gaseous stream across the supports of the biomass.

2. A biofilter according to claim 1, wherein the straight passage supports are hollow tubes.

3. A biofilter according to claim 1, wherein the straight passage supports are made using extruded activated carbon.

4. A biofilter according to claim 1, wherein the straight passage supports are made of ceramics.

5. A method of biodegrading and/or biotransforming volatile compounds, said method comprising the steps of:

   (a) maintaining a biofilter according to claim 1 in a moist state; and
   (b) passing a gas stream containing said volatile compounds over said biofilter countercurrent to a liquid stream for sufficient time to at least partially biotransform or biodegrade said volatile compounds.

6. A method according to claim 5, wherein the volatile compounds subjected to biotransformation are sulphur oxides and/or nitrogen oxides.

7. A method according to claim 5 or claim 6, wherein the volatile compounds subjected to biodegradation or biotransformation comprise an organic compound.

**Patentansprüche**

1. Biofilter mit

   a) einem Bett mit benetzbaren geraden Durchgangsträgern mit daran haftender Biomasse, wobei diese Durchgangsträger unter mehreren hohlen Röhren, Fasern, extrudiertem Material mit mehreren geraden Kanaldurchgängen, die vertikal durch das extrudierte Material gehen, oder gewellten Platten, die so angeordnet sind, daß sie vertikale gerade Durchgänge bilden, ausgewählt sind,

   b) einem Einlaß für den Zugang eines gasförmigen Stromes in das Biofilter und

   c) einem Einlaß für einen Flüssigkeitsstrom, der in solcher Weise angeordnet ist, daß der Flüssigkeitsstrom im Gegenstrom zu dem gasförmigen Strom quer zu den Tägern der Biomasse geht.

2. Biofilter nach Anspruch 1, bei dem die geraden Durchgangsträger hohle Röhren sind.

3. Biofilter nach Anspruch 1, bei dem die geraden Durchgangsträger unter Verwendung von extrudiertem aktiviertem Kohlenstoff hergestellt sind.

4. Biofilter nach Anspruch 1, bei dem die geraden Durchgangsträger aus Keramikmaterialien hergestellt sind.

5. Verfahren zum biologischen Abbau und/oder zur biologischen Umwandlung flüchtiger Verbindungen mit den Stufen, in denen man

   a) ein Biofilter nach Anspruch 1 in einem feuchten Zustand hält und

   b) einen die flüchtigen Verbindungen enthaltenden Gasstrom im Gegenstrom zu einem Flüssigkeitsstrom ausreichend lange, um die flüchtigen Verbindungen wenigstens teilweise biologisch umzuwandeln oder biologisch abzubauen, über das Biofilter führt.

6. Verfahren nach Anspruch 5, bei dem die flüchtigen Verbindungen, die einer biologischen Umwandlung unterzogen werden, Schwefeloxide und/oder Stickstoffoxide sind.

7. Verfahren nach Anspruch 5 oder 6, bei dem die flüchtigen Verbindungen, die einem biologischen Abbau oder einer biologischen Umwandlung unterzogen werden, eine organische Verbindung umfassen.

**Revendications**

1. Biofiltre comprenant :

   (a) un lit comportant des supports de passages droits mouillables avec une biomasse adhérant à ceux-ci, lesdits supports de passages étant sélectionnés parmi une pluralité de tubes creux, fibres, matériau extrudé avec de multiples passages de canaux droits s'étendant verticalement dans ledit matériau extrudé ou des plaques cannelées agencées de manière à former des passages droits verticaux ;

(b) une entrée pour admission d'un flux gazeux dans le biofiltre ; et

(c) une entrée pour un flux liquide positionnée de manière que le flux liquide s'écoule à contre-courant du flux gazeux en travers des supports de la biomasse.

2.  Biofiltre selon la revendication 1, dans lequel les supports de passages droits sont des tubes creux.

3.  Biofiltre selon la revendication 1, dans lequel les supports de passages droits sont réalisés en utilisant du charbon actif extrudé.

4.  Biofiltre selon la revendication 1, dans lequel les supports de passages droits sont constitués de céramique.

5.  Procédé de biodégradation et/ou biotransformation de composés volatils, ledit procédé comprenant les phases consistant à :

(a) maintenir un biofiltre selon la revendication 1 dans un état humide ; et

(b) faire passer un flux de gaz contenant lesdits composés volatils sur ledit biofiltre à contre-courant d'un flux liquide pendant une durée suffisante pour au moins partiellement biotransformer ou biodégrader lesdits composés volatils.

6.  Procédé selon la revendication 5, dans lequel les composés volatils soumis à une biotransformation sont des oxydes de soufre et/ou des oxydes d'azote.

7.  Procédé selon la revendication 5 ou la revendication 6, dans lequel les composés volatils soumis à une biodégradation ou une biotransformation comprennent un composé organique.

FIG. 1

FIG. 2A

FIG. 2B

FIG. 3

FIG. 3A

FIG. 3B

FIG. 4